# EUROPÄISCHE PATENTSCHRIFT

(11) **EP 3 455 000 B1**
(45) Veröffentlichungstag und Bekanntmachung des Hinweises auf die Patenterteilung: **14.10.2020**
(21) Anmeldenummer: 17722459.9
(22) Anmeldetag: 10.05.2017
(51) Int. Cl.: B07C 5/342

(54) **VERFAHREN UND VORRICHTUNG FÜR DAS LEGIERUNGSABHÄNGIGE SORTIEREN VON METALLSCHROTT, INSBESONDERE ALUMINIUMSCHROTT**
METHOD AND DEVICE FOR ALLOY BASED SORTING OF SCRAP METAL, IN PARTICULAR OF SCRAP ALUMINIUM
PROCÉDÉ ET DISPOSITIF DE TRI DE DÉCHETS MÉTALLIQUES SELON L'ALLIAGE, EN PARTICULIER POUR LES DÉCHETS D'ALUMINIUM

(30) Priorität: 11.05.2016 DE 102016108745
(43) Veröffentlichungstag der Anmeldung: 20.03.2019
(73) Patentinhaber: Hydro Aluminium Rolled Products GmbH, 41515 Grevenbroich (DE)
(72) Erfinder: GILLNER, Ronald, 53913 Swisttal (DE); BAUERSCHLAG, Nils Robert, 52072 Aachen (DE)
(74) Vertreter: Cohausz & Florack
(86) Internationale Anmeldenummer: PCT/EP2017/061149
(87) Internationale Veröffentlichungsnummer: WO 2017/194585

(56) Entgegenhaltungen:
- EP-A1- 0 293 983
- EP-A1- 1 416 265
- DE-A1-102012 015 812

## Beschreibung

Die Erfindung betrifft ein Verfahren und eine Vorrichtung für das legierungsabhängige Sortieren von Metallschrott, insbesondere Aluminiumschrott.

Im Stand der Technik erfolgt das Sortieren bzw. Recyceln von Aluminium über mehrere Verfahrensschritte. Diese umfassen in der Regel das Sammeln der unterschiedlichen Aluminiumschrotte, eine mechanische Aufbereitung der Schrotte und eine anschließende metallurgische Verwertung der Schrotte.

Für ein ressourceneffizientes Recycling sollte die mechanische Aufbereitung der Schrotte ein Aluminiumschrottprodukt erzeugen, das den qualitativen Ansprüchen des metallurgischen Verwertungsweges entspricht. Hierzu werden im Stand der Technik unterschiedliche Aufbereitungsschritte durchgeführt, die allerdings nur eine begrenzte Sortierung in Qualitäten bzw. Legierungszusammensetzungen der Schrotte erlaubt.

Die mechanische Aufbereitung erfolgt in der Regel über eine ein- oder mehrstufige Zerkleinerung der Schrotte, der sich dann diverse Sortierschritte anschließen. Die Sortierschritte können beispielsweise eine Trennung von Eisen und NE-Metallen über Magnetscheider, Windsichtung, Wirbelstromscheidung, sensorgestützte Sortierungen zum Beispiel mittels Röntgentransmission oder -fluoreszens, Induktion, laserinduzierte Plasmaspektroskopie (LIBS) oder Nahinfrarotanalyse (NIR) bewirken. Die verfahrenstechnische Kombination dieser Sortierschritte erlaubt das Sortieren der Schrotte in unterschiedliche Aluminiumqualitäten, d.h. insbesondere abhängig von ihrer Legierungszusammensetzung.

Um unterschiedliche Aluminiumlegierungen legierungsspezifisch sortieren zu können, müssen ein oder mehrere Legierungselemente der einzelnen Schrottfragmente bestimmt werden. Hierzu werden typischerweise Systeme für laserinduzierte Plasmaspektroskopie (LIBS) oder Röntgenfluoreszenz (XRF) eingesetzt. Die mit diesen Systemen erzeugten Analyseergebnisse werden mit vorgegebenen Legierungszusammensetzungen verglichen und die jeweiligen Schrottfragmente der passenden Legierungszusammensetzung zugeordnet. Wird beispielsweise bei der Analyse eines Schrottfragments ein Mg-Gehalt von 5% ermittelt, so wird dieses Schrottfragment einer Mg5-Legierung zugeordnet.

Es wurde jedoch festgestellt, dass trotz der Analyse der Schrottfragmente nur eine mäßig gute legierungsspezifische Sortierung von Aluminiumschrotten erreicht werden kann.

Aus der DE 10 2012 015 812 A1 ist ein Verfahren zur Aufarbeitung von Stahlschrotten bekannt, bei dem die Stahlschrotte mit einer Flüssigkeit zur Ablösung von Oberflächenbeschichtungen behandelt werden, bevor eine spektroskopische Analyse der Stahlschrottzusammensetzung erfolgt. Aus der EP 0 293 983 A1 ist eine LIBS-Analyse von Metallteilchen zur Gewinnung eines Sortiersignals bekannt. Aus der EP 1 416 265 A1 ist ein System zur schnellen Schrottteilchensortierung durch optische Analyse laserinduzierter Plasmen bekannt.

Vor diesem Hintergrund liegt der vorliegenden Erfindung die Aufgabe zu Grunde, ein Verfahren und eine Vorrichtung für das Sortieren von Metallschrott, insbesondere Aluminiumschrott, bereitzustellen, die eine bessere Sortierung der Schrotte erlauben.

Diese Aufgabe wird erfindungsgemäß zumindest teilweise gelöst durch Verfahren für das legierungsabhängige Sortieren von Metallschrott, insbesondere Aluminiumschrott, bei dem an einem Schrottfragment eine Zusammensetzungsanalyse durchgeführt wird, wobei mittels einer Messung an dem Schrottfragment eine Oberflächenzusammensetzungsinformation über die lokale Zusammensetzung in einem Oberflächenbereich des Schrottfragments bestimmt wird, und bei dem dem Schrottfragment abhängig von der mittels Messung bestimmten Oberflächenzusammensetzungsinformation und einer vorgegebenen Zuordnungsvorschrift eine zugehörige Volumenzusammensetzungsinformation über die Zusammensetzung des Schrottfragments im Volumen zugeordnet wird.

Bei dem Verfahren wird an einem Schrottfragment eine Zusammensetzungsanalyse durchgeführt, bei der mittels einer Messung an dem Schrottfragment eine

Oberflächenzusammensetzungsinformation über die lokale Zusammensetzung in einem Oberflächenbereich des Schrottfragments bestimmt wird.

Die Oberflächenzusammensetzungsinformation umfasst insbesondere Werte und/oder Wertebereiche für den Gehalt von Legierungselementen, beispielsweise eines oder mehrerer aus der Gruppe Mg, Mn, Si oder Fe.

Die Oberflächenzusammensetzungsinformation umfasst Informationen über die lokale Zusammensetzung in einem Oberflächenbereich des Schrottfragments. Unter einem Oberflächenbereich des Schrottfragments wird vorliegend ein oberflächennahes Volumen des Schrottfragments verstanden. Oberflächensensitive Messverfahren, wie beispielsweise LIBS oder XRF, haben eine begrenzte Analysetiefe und analysieren die Zusammensetzung eines Materials daher nur in einem oberflächennahen Volumen zwischen der Oberfläche des Schrottfragments und der Analysetiefe. Die Oberflächenzusammensetzungsinformation entspricht damit einer Zusammensetzungsinformation, die mit einem solchen oberflächensensitiven Messverfahren gemessen werden kann.

Im Stand der Technik werden die mit einem solchen Analyseverfahren, beispielsweise durch LIBS oder XRF, ermittelten Zusammensetzungsinformationen zum Abgleich mit vorgegebenen Legierungszusammensetzungen verwendet, um die Schrottfragmente eine dieser Legierungszusammensetzungen zuzuordnen.

Im Rahmen der Erfindung wurde jedoch erkannt, dass dieses Vorgehen zu Fehlinterpretationen führen kann, da die verwendeten Analyseverfahren Analyseergebnisse liefern, die von der tatsächlichen gesamten Zusammensetzung des Schrottfragments abweichen.

Aufgrund von Seigerungs- und Diffusionseffekten im Material des Schrottfragments kann die oberflächennahe Zusammensetzung nämlich zum Teil deutlich von der tatsächlichen Zusammensetzung des gesamten Schrottfragments im Volumen abweichen. Insbesondere führen die Seigerungs- und Diffusionseffekte zu einer elementspezifischen Anreicherung einzelner Legierungselemente an der Oberfläche des Schrottfragments. Erschwerend kommt noch hinzu, dass die Seigerungs- und Diffusionseffekte für einzelne Elemente unterschiedlich ausgeprägt sind.

Beispielsweise kommt es bei niedriglegierten Aluminiumlegierungen, bei der der Mg-Gehalt im Volumen bei 0,5 Gew.-% liegt, zu einer oberflächennahen Anreicherung des Mg, so dass der Mg-Gehalt an der Oberfläche um einen Faktor 10 über dem eigentlichen Mg-Gehalts im Volumen liegt.

Die oberflächennahe Analyse der Zusammensetzung des Schrottfragments liefert dann ein Ergebnis, das mit der eigentlichen Zusammensetzung des Schrottfragments nicht übereinstimmt und somit zu Fehlinterpretationen führt. Beispielsweise würde die niedriglegierte Legierung Mg0,5 wegen des gemessenen hohen Mg-Gehalts an der Oberfläche fälschlicherweise einer höherlegierten Legierung Mg5 zugeordnet und entsprechend falsch einsortiert.

Bei Laser-gestützten Verfahren lässt sich die Eindringtiefe zwar grundsätzlich durch Erhöhung der Laserleistung erhöhen. Allerdings dringt ab einer bestimmten Eindringtiefe nicht mehr ausreichend Licht aus dem durch den Laserstrahl erzeugten Krater in der Schrottfragmentoberfläche zur LIBS-Analyse, so dass die Analysetiefe dennoch begrenzt ist. Weiterhin ist der durch den Laserstrahl erzeugte Krater an der Schrottfragmentoberfläche typischerweise breiter als in der Tiefe. So kann der Krater beispielsweise kegelförmig ausgebildet sein. Dies führt dazu, dass die LIBS-Analyse durch das oberflächennahe Signal dominiert wird, da aus diesem Bereich das meiste Material verdampft wird.

Auf Grundlage dieser Erkenntnis wird dem analysierten Schrottfragment bei dem vorliegend beschriebenen Verfahren abhängig von der mittels Messung bestimmten Oberflächenzusammensetzungsinformation und einer vorgegebenen Zuordnungsvorschrift eine zugehörige Volumenzusammensetzungsinformation über die Zusammensetzung des Schrottfragments im Volumen zugeordnet. Anstatt das Schrottfragment allein über das gemessene Ergebnis der Zusammensetzungsanalyse einer Legierung zuzuordnen, was im Stand der Technik zu den zuvor beschriebenen Fehlinterpretationen führt, wird dem Schrottfragment also eine der durch Messung ermittelten Oberflächenzusammensetzungsinformation über die Zuordnungsvorschrift zugeordnete Volumenzusammensetzungsinformation zugeordnet, die die tatsächliche Zusammensetzung des Schrottfragments im Volumen charakterisiert.

Insbesondere unterscheiden sich die Oberflächenzusammensetzungsinformation und die dieser Oberflächenzusammensetzungsinformation zugeordnete Volumenzusammensetzungsinformation zumindest teilweise voneinander.

Beispielsweise kann einer Oberflächenzusammensetzungsinformation, die einen Mg-Gehalt von 5% angibt, eine Volumenzusammensetzungsinformation mit einem Mg-Gehalt von 0,5% zugeordnet werden. Dadurch werden die Seigerungs- und Diffusionseffekte im Schrottbauteil, die zu einer oberflächennahen Erhöhung des Mg-Gehalts führen, bei der Zuordnung berücksichtigt, so dass das Schrottbauteil in geeigneter Weise weiter verwendet, insbesondere sortiert werden kann.

Auf diese Weise kann die Zuverlässigkeit und Effizienz bei der Sortierung von Metall-, insbesondere Aluminiumschrotten verbessert werden. Insbesondere werden Analyseergebnisse für die Schrottfragmente erzielt, bei denen elementspezifische Seigerungs- und Diffusionseffekte berücksichtigt werden.

In Abhängigkeit der Eingangsschrottzusammensetzung und der Anzahl an gemessenen Legierungselementen kann auf diese Weise eine legierungsspezifische Bewertung bzw. Zuordnung der einzelnen Schrottfragmente erfolgen. Diese kann anschließend beispielsweise als Sortierkriterium genutzt werden, um Schrottfragmente aus verschiedenen Legierungen voneinander zu trennen. Weiterhin ist es möglich, die Analyseergebnisse für einzelne Elemente als Sortierkriterium zu nutzen, beispielsweise um Schrottfragmente mit einem besonders hohen oder besonders niedrigen Gehalt eines bestimmten Legierungselements auszusortieren.

Die Volumenzusammensetzungsinformation umfasst insbesondere Werte und/oder Wertebereiche für den Gehalt von Legierungselementen, beispielsweise eines oder mehrere aus der Gruppe Mg, Mn, Si oder Fe.

Die oben genannte Aufgabe wird erfindungsgemäß weiterhin zumindest teilweise gelöst durch eine Vorrichtung für die Sortierung von Metallschrotten, insbesondere Aluminiumschrotten, vorzugsweise zur Durchführung des zuvor beschriebenen Verfahrens, mit einer Fördereinrichtung, eingerichtet zur Förderung einer Menge Schrottfragmente, mit einer Analyseeinrichtung, eingerichtet zur Durchführung von Zusammensetzungsanalysen von auf der Fördereinrichtung geförderten Schrottfragmenten, wobei eine Zusammensetzungsanalyse eines Schrottfragments die Bestimmung einer Oberflächenzusammensetzungsinformation über die lokale Zusammensetzung in einem Oberflächenbereich des Schrottfragments mittels einer Messung umfasst, und mit einer Steuerungseinrichtung, die dazu eingerichtet ist, den mit der Analyseeinrichtung analysierten Schrottfragmenten abhängig von der mittels Messung bestimmten Oberflächenzusammensetzungsinformation und einer vorgegebenen Zuordnungsvorschrift jeweils eine zugehörige Volumenzusammensetzungsinformation über die Zusammensetzung des Schrottfragments im Volumen zuzuordnen.

Die Vorrichtung weist eine Fördereinrichtung auf, die zur Förderung einer Menge Schrottfragmente eingerichtet ist. Bei der Fördereinrichtung kann es sich beispielsweise um ein Förderband handeln. Mit der Fördereinrichtung können die Schrottfragmente durch die Vorrichtung transportiert werden, insbesondere von einem Materialeinzug zu der Analyseeinrichtung, so dass die am Materialeinzug auf die Fördereinrichtung gegebenen Schrottfragmente zur Analyseeinrichtung transportiert und dort analysiert werden können.

Die Vorrichtung weist weiterhin eine Analyseeinrichtung auf, die zur Durchführung von Zusammensetzungsanalysen von auf der Fördereinrichtung geförderten Schrottfragmenten eingerichtet ist. Handelt es sich bei der Fördereinrichtung beispielsweise um ein Förderband, so kann die Analyseeinrichtung insbesondere ein Analysegerät aufweisen, das oberhalb des Förderbands angeordnet ist, um die auf dem Förderband transportierten Schrottfragmente zu untersuchen.

Die mit der Analyseeinrichtung durchführbare Zusammensetzungsanalyse eines Schrottfragments umfasst die Bestimmung einer Oberflächenzusammensetzungsinformation über die lokale Zusammensetzung in einem Oberflächenbereich des Schrottfragments mittels einer Messung. Mit der Analyseeinrichtung wird demnach nicht die gesamte Zusammensetzung des Schrottfragments erfasst, sondern die Zusammensetzung des Schrottfragments an dessen Oberfläche. Mit anderen Worten erfolgt mit der Analyseeinrichtung eine oberflächliche Zusammensetzungsanalyse des Schrottbauteils, vorzugsweise mit einer vorbestimmten Analysetiefe.

Die Vorrichtung umfasst weiterhin eine Steuerungseinrichtung. Die Steuerungseinrichtung kann insbesondere einen Mikroprozessor und einen damit verbundenen Speicher aufweisen, der Befehle enthält, deren Ausführung auf dem Mikroprozessor eine Verarbeitung von Daten und/oder die Steuerung der Vorrichtung veranlasst.

Die Steuerungseinrichtung ist dazu eingerichtet, dem mit der Analyseeinrichtung analysierten Schrottfragment abhängig von der mittels Messung bestimmten Oberflächenzusammensetzungsinformation und einer vorgegebenen Zuordnungsvorschrift eine zugehörige Volumenzusammensetzungsinformation über die Zusammensetzung des Schrottfragments im Volumen zuzuordnen. Beispielsweise kann in einem Speicher der Steuerungseinrichtung eine Funktion gespeichert sein, die aus Werten für die Gehalte von Legierungskomponenten aus der Oberflächenzusammensetzungsinformation durch Anwendung definierter Rechenregeln Werte für die Gehalte von Legierungskomponenten für die Volumenzusammensetzungsinformation berechnet. Die Rechenregeln sind insbesondere derart definiert, dass sie eine Erhöhung bzw. Erniedrigung von Legierungselementen an der Oberfläche aufgrund von Seigerungs- und Diffusionseffekten kompensiert. Beispielsweise kann die Rechenregel eine Division eines Mg-Gehalts aus der Oberflächenzusammensetzungsinformation um denjenigen Faktor umfassenerhöht, um den der Mg-Gehalt an der Oberfläche im Verhältnis zum Volumen aufgrund von Diffusions- und Seigerungseffekten erhöht ist, um dadurch einen Mg-Gehalt einer zuzuordnenden Volumenzusammensetzungsinformation zu bestimmen.

Im Folgenden werden verschiedene Ausführungsformen der Vorrichtung und des Verfahrens beschrieben, wobei die einzelnen Ausführungsformen jeweils sowohl für die Vorrichtung, als auch für das Verfahren angewendet werden können. Weiterhin sind die einzelnen Ausführungsformen beliebig untereinander kombinierbar.

Bei einer ersten Ausführungsform des Verfahrens wird eine Menge von Schrottfragmenten bereitgestellt, es wird für mehrere Schrottfragmente aus der Menge von Schrottfragmenten jeweils eine Zusammensetzungsanalyse durchgeführt, wobei mittels einer Messung an dem jeweiligen Schrottfragment eine Oberflächenzusammensetzungsinformation über die lokale Zusammensetzung in einem Oberflächenbereich des jeweiligen Schrottfragments bestimmt wird, und es wird dem jeweiligen Schrottfragment abhängig von der mittels Messung bestimmten Oberflächenzusammensetzungsinformation und einer vorgegebenen Zuordnungsvorschrift eine zugehörige Volumenzusammensetzungsinformation über die Zusammensetzung des jeweiligen Schrottfragments im Volumen zugeordnet. Auf diese Weise kann eine Menge Schrottfragmente in schneller und zuverlässiger Weise in Schrottfragment-genauer (d.h. Einzelkorn-genauer) Auflösung analysiert werden. Insbesondere lässt sich eine Menge Schrott auf diese Weise zuverlässig charakterisieren. Beispielsweise kann auf diese Weise eine genaue Zusammensetzung der Menge Schrott bestimmt werden. Eine auf diese Weise bestimmte Zusammensetzung kann dann beispielsweise für Ofenchargier-Programme oder zur Kontrolle von Schrottlieferungen genutzt werden. Weiterhin können die Schrottfragmente anhand der zugeordneten Volumenzusammensetzungsinformationen sortiert werden.

Die Menge von Schrottfragmenten kann beispielsweise über ein Förderband bereitgestellt werden, über das die Schrottfragmente zu einem Ort für die Durchführung der Zusammensetzungsanalyse transportiert werden. Vorzugsweise werden die Schrottfragmente vor der Durchführung der Zusammensetzungsanalyse vereinzelt, so dass die einzelnen Schrottfragmente räumlich voneinander getrennt werden, beispielsweise nacheinander auf einem Förderband transportiert werden. Dies erleichtert die Analyse der einzelnen Schrottfragmente und die Zuordnung der jeweiligen Volumenzusammensetzungsinformation zu den einzelnen Schrottfragmenten. Entsprechend weist die Vorrichtung vorzugsweise eine Vereinzelungseinrichtung auf, die zur Vereinzelung von Schrottfragmenten vor deren Analyse eingerichtet ist.

Bei einer weiteren Ausführungsform des Verfahrens wird das Schrottfragment abhängig von der zugeordneten Volumenzusammensetzungsinformation sortiert. Wird eine Menge von Schrottfragmenten bereitgestellt, so werden entsprechend die Schrottfragmente aus der Menge von Schrottfragmenten abhängig von der jeweils zugeordneten Volumenzusammensetzungsinformation sortiert. Bei einer entsprechenden Ausführungsform der Vorrichtung umfasst diese eine Sortiereinrichtung, die dazu eingerichtet ist, Schrottfragmente abhängig von der den Schrottfragmenten durch die Steuerungseinrichtung jeweils zugeordneten Volumenzusammensetzungsinformation zu sortieren.

Die bei dem Verfahren bestimmte Volumenzusammensetzungsinformation eines Schrottfragments, die die tatsächliche Volumenzusammensetzung des Schrottfragments erheblich zuverlässiger charakterisiert als die gemessene Oberflächenzusammensetzungsinformation, kann auf diese Weise unmittelbar eingesetzt werden, um das Schrottfragment legierungsspezifisch zu sortieren. Insbesondere kann auf diese Weise eine Menge Schrottfragmente, die Schrottfragmente aus mehreren Legierungen enthält, sortiert werden.

Die Sortiereinrichtung kann insbesondere eine Materialzuführung aufweisen, über die die Schrottfragmente zur Sortiereinrichtung transportiert werden sowie einen Sortierer, der die über die Materialzuführung zugeführten Schrottfragmente abhängig von der den Schrottfragmenten jeweils zugeordneten Volumenzusammensetzungsinformation einer von mindestens zwei vorgesehenen Materialabführungen zuordnet, mit denen die sortierten Schrottfragmente von der Sortiereinrichtung abgeführt werden.

Bei einer weiteren Ausführungsform des Verfahrens wird dem Schrottfragment abhängig von der mittels Messung bestimmten Oberflächenzusammensetzungsinformation und einer vorgegebenen Zuordnungsvorschrift eine zugehörige Volumenzusammensetzungsinformation zugeordnet, indem abhängig von der mittels Messung bestimmten Oberflächenzusammensetzungsinformation und der vorgegebenen Zuordnungsvorschrift eine Volumenzusammensetzungsinformation aus einer Mehrzahl vorgegebener Volumenzusammensetzungsinformationen ausgewählt wird.

Beispielsweise kann in einem Speicher der Steuerungseinrichtung eine Zuordnungsvorschrift gespeichert sein, die mehreren vorgegebenen Legierungsbereichen für die Oberflächenzusammensetzungsinformation jeweils einen Legierungsbereich für die Volumenzusammensetzungsinformation zuordnet. Die Zuordnung zwischen einem Legierungsbereich für die Oberflächenzusammensetzungsinformation und einem Legierungsbereich für die Volumenzusammensetzungsinformation ist insbesondere jeweils so gewählt, dass Schrottfragmente aus einer Legierung im Legierungsbereich für die Volumenzusammensetzungsinformation bei Analyse mit der Analysevorrichtung eine Oberflächenzusammensetzungsinformation im jeweils zugeordneten Legierungsbereich für die Oberflächenzusammensetzungsinformation zeigen. Für eine durch Messung bestimmte Oberflächenzusammensetzungsinformation kann dann derjenige vorgegebene Legierungsbereich für die Volumenzusammensetzungsinformation ausgewählt werden, der demjenigen vorgegebenen Legierungsbereich für die Oberflächenzusammensetzungsinformation zugeordnet ist, in den die durch Messung ermittelte Oberflächenzusammensetzungsinformation fällt

Zu diesem Zweck wird die durch Messung ermittelte Oberflächenzusammensetzungsinformation insbesondere mit den einzelnen Legierungsbereichen für die Oberflächenzusammensetzungsinformation verglichen. Entsprechend ist bei einer weiteren Ausführungsform des Verfahrens den vorgegebenen Volumenzusammensetzungsinformationen jeweils eine vorgegebene zugehörige Oberflächenzusammensetzungsinformation zugeordnet und die Auswahl der Volumenzusammensetzungsinformation aus der Mehrzahl vorgegebener Volumenzusammensetzungsinformationen erfolgt durch einen Vergleich der mittels Messung bestimmten Oberflächenzusammensetzungsinformation mit den vorgegebenen Oberflächenzusammensetzungsinformationen.

Der ausgewählte Legierungsbereich für die Volumenzusammensetzungsinformation kann dann als Volumenzusammensetzungsinformation dem entsprechenden Schrottfragment zugeordnet werden.

Die Zuordnungsvorschrift bzw. die Legierungsbereiche für die Oberflächenzusammensetzungsinformation, die Legierungsbereiche für die Volumenzusammensetzungsinformation und die jeweiligen Zuordnungen zueinander können beispielsweise vor Durchführung des vorliegend beschriebenen Verfahrens mittels einer schichtweisen Zusammensetzungsanalyse von typischen Schrottfragmenten durchgeführt werden, beispielsweise mittels Glimmentladungsspektroskopie (GDOES).

Die GDOES-Analyse liefert eine tiefenabhängig Zusammensetzung des Schrottfragments, die es erlaubt festzustellen, welche Volumenzusammensetzungen sich aufgrund von Seigerungs- und Diffusionseffekten in welchen Oberflächenzusammensetzungen manifestieren. Das Ergebnis dieser Analyse erlaubt es, einer gemessenen Oberflächenzusammensetzungsinformation eine zugehörige Volumenzusammensetzungsinformation zuzuordnen. Insbesondere können die Ergebnisse dieser Analyse zur Definition der Zuordnungsvorschrift bzw. von Legierungsbereichen für die Oberflächenzusammensetzungsinformation, Legierungsbereichen für die Volumenzusammensetzungsinformation und von jeweiligen Zuordnungen zueinander verwendet werden.

Auf diese Weise können die Ergebnisse einer recht aufwändigen GDOES-Analyse verwendet werden, um die Ergebnisse einer schnelleren Analyse der Schrottfragmente bei dem hier beschriebenen Verfahren, insbesondere mittels LIBS oder XRF, zu interpretieren. Insbesondere können die Seigerungs- und Diffusionseffekte von Aluminiumlegierungen über die GDOES hinreichend genau bestimmt werden. Die Analyseergebnisse, die die Elementgehalte in unterschiedlichen Oberflächentiefen darstellen, können dann über die Definition einer entsprechenden Zuordnungsvorschrift die Datenbank für die Bewertung der Messergebnisse zum Beispiel eines LIBS-Systems darstellen, das dann für die anschließende Sortierung eingesetzt wird.

Durch die dadurch erreichte Zuordnung von oberflächlichen Messergebnissen eines LIBS-Systems (zur Sortierung) zu den Ergebnissen einer GDOES-Analyse, können signifikant genauere Elementanalysen einzelner Schrottfragmente erreicht werden, wodurch eine legierungsscharfe Sortierung ermöglicht wird.

Die Zuordnungsvorschrift kann auch Korrelationen zwischen einzelnen Legierungselementen und deren spezifischen Seigerungs- und Diffusionseffekten heranziehen, um die mittels Messung bestimmte Oberflächenzusammensetzungsinformation einer zugehörigen Volumenzusammensetzungsinformation zuzuordnen. Insbesondere kann die Zuordnungsvorschrift eine Zuordnung abhängig von einem Verhältnis der Gehalte zweier Legierungselemente vornehmen, beispielsweise das Verhältnis des Mg-Gehalts zum Mn-Gehalt einer Aluminiumlegierung. Auf diese Weise kann zum Beispiel das mit einem LIBS-System ermittelte Verhältnis der Gehalte aus der durch den Laser des LIBS-Systems erfassten Analysetiefe praktisch als Fingerabdruck verwendet werden, um diese Oberflächenzusammensetzungsinformation einer bestimmten Legierung, insbesondere einem Analysewert aus einer GDOES-Messung und damit einer Volumenzusammensetzungsinformation über die Zusammensetzung des Schrottfragments im Volumen zuzuordnen.

Bei einer weiteren Ausführungsform des Verfahrens wird bei der Zusammensetzungsanalyse eine Oberflächenzusammensetzungsinformation über die lokale Zusammensetzung in einem Oberflächenbereich des Schrottfragments bestimmt, wobei sich der Oberflächenbereich von der Oberfläche des Schrottfragments bis in eine vorbekannte oder vorgegebene Tiefe (Analysetiefe), beispielsweise bis in eine Tiefe im Bereich vom 1 - 100 µm, insbesondere 1 - 10 µm, erstreckt. Bei der Verwendung von LIBS kann die Tiefe beispielsweise durch die Laserleistung und/oder die Wahl eines bestimmten Lasertyps eingestellt werden. Durch Festlegen des Lasertyps und/oder der Laserleistung ist die Analysetiefe damit fest und somit vorbekannt. Entsprechend wird die Zuordnungsvorschrift vorzugsweise abhängig von einer vorbekannten oder vorgegebenen Analysetiefe ausgewählt.

Durch die Wahl des Lasertyps, der Laserleistung und/oder der verwendeten Optik für den Laser lassen sich auch der Footprint des Laserstrahls, d.h. die Größe des Spots des Laserstrahls auf der Schrottfragmentoberfläche, und/oder die Kraterform des vom Laserstrahl in die Schrottfragmentoberfläche eingebrachten Kraters vorgeben. Entsprechend wird die Zuordnungsvorschrift vorzugsweise abhängig vom vorbekannten oder vorgegebenen Footprint und/oder einer vorbekannten oder vorgegebenen Kraterform ausgewählt. Es ist beispielsweise denkbar, dass ein Laserstrahl einen zylindrischen, einen kegelförmigen oder einen kugelförmigen Krater in der Schrottfragmentoberfläche erzeugt. Bei einem zylindrischen Krater tragen die Signale aus verschiedenen Tiefen ungefähr gleich zum Gesamtergebnis der LIBS-Messung bei. Bei einem kegelförmigen Krater, der sich in die Tiefe verjüngt, dominieren die oberflächennahen Bereiche des Kraters das Gesamtergebnis der LIBS-Messung. Durch Berücksichtigung der Kraterform kann daher eine bessere Zuordnung zur Volumenzusammensetzungsinformation erreicht werden.

Dadurch dass die Analysetiefe vorbekannt ist, kann eine zuverlässigere Zuordnung einer Volumenzusammensetzungsinformation zu der mittels Messung bestimmten Oberflächenzusammensetzungsinformation erfolgen. Wird zur Definition der Zuordnungsvorschrift beispielsweise wie zuvor beschrieben eine GDOES-Analyse durchgeführt, die die tiefenabhängige Zusammensetzung von Schrottfragmenten liefert, so kann die bei einer LIBS-Messung ermittelte Oberflächenzusammensetzungsinformation dadurch bestimmt werden, dass die tiefenabhängige Zusammensetzung von der Oberfläche bis zur vorbekannten Analysetiefe gemittelt bzw. integriert wird. Durch eine gewichtete Mittelung bzw. Integration kann die Kraterform mitberücksichtigt werden. Auf diese Weise lässt sich das Messergebnis der LIBS-Messung für ein Schrottfragment bekannter Zusammensetzung voraussagen und somit eine geeignete Zuordnungsvorschrift definieren.

Bei einer weiteren Ausführungsform des Verfahrens umfasst die Zusammensetzungsanalyse eine spektroskopische Analyse, insbesondere eine laserinduzierte Plasmaspektroskopie (LIBS) oder eine Röntgenfluoreszenzanalyse (XRF). Bei einer entsprechenden Ausführungsform der Vorrichtung umfasst die Analyseeinrichtung ein spektroskopisches Analysegerät, insbesondere ein Analysegerät für laserinduzierte Plasmaspektroskopie (LIBS) oder Röntgenfluoreszenzanalyse (XRF). Durch die spektroskopische Analyse lässt sich auf schnelle Weise eine Oberflächenzusammensetzungsinformation eines Schrottelements messen. Die laserinduzierte Plasmaspektroskopie und die Röntgenfluoreszenzanalyse haben sich als besonders geeignete Verfahren herausgestellt, um die Oberflächenzusammensetzungsinformation schnell und zuverlässig zu messen. Da beide Verfahren oberflächensensitiv sind, d.h. die Zusammensetzung nur im Oberflächenbereich des Schrottelements bestimmen können, ist eine unmittelbare Sortierung abhängig von einer LIBS- oder XRF-Messung problematisch. Durch das vorliegend beschriebene Verfahren bzw. die vorliegend beschriebene Vorrichtung können LIBS- und XRF-Messungen nun für eine zuverlässige Legierungszuordnung von Schrottfragmenten eingesetzt werden.

Bei einer weiteren Ausführungsform des Verfahrens umfasst die durch Messung bestimmte Oberflächenzusammensetzungsinformation Werte für die Gehalte von mindestens zwei Legierungskomponenten des Schrottfragments. Beispielsweise kann die Oberflächenzusammensetzungsinformation Werte für die Gehalte von zwei oder mehr der Legierungselemente Mg, Mn, Si oder Fe enthalten. Es wurde festgestellt, dass die Zuordnung einer Volumenzusammensetzungsinformationen zu einer Oberflächenzusammensetzungsinformation zuverlässiger ist, wenn mehr als ein Legierungskomponente des Schrottfragments analysiert wird. Anderseits ist typischerweise eine Zahl von maximal vier Legierungskomponenten ausreichend, um eine die Oberflächenzusammensetzungsinformation zuverlässig zuzuordnen, so dass die Oberflächenzusammensetzungsinformation vorzugsweise Werte für die Gehalte von maximal vier Legierungskomponenten des Schrottfragments umfasst. Dadurch kann die Auswertung der mit der Analyseeinrichtung ermittelten Messwerte vereinfacht und in kürzerer Zeit durchgeführt werden.

Bei einer weiteren Ausführungsform des Verfahrens wird eine Menge Schrottfragmente legierungsabhängig sortiert, indem die Zusammensetzungsanalyse und Sortierung für die einzelnen Schrottfragmente aus der Menge durchgeführt wird. Demnach werden mehrere Schrottfragmente einer Menge abhängig von der jeweils bestimmten Zusammensetzungsanalyse legierungsabhängig sortiert.

Bei einer weiteren Ausführungsform werden die Schrottfragmente vereinzelt, bevor an den Schrottfragmenten eine Zusammensetzungsanalyse durchgeführt wird. Bei einer entsprechenden Ausführungsform der Vorrichtung umfasst diese eine Vereinzelungseinrichtung, die dazu eingerichtet ist, Schrottfragmente zu vereinzeln, bevor sie der Analyseeinrichtung zugeführt werden. Durch die Vereinzelung weisen die Schrottfragmente eine vorgegebene Reihenfolge auf, mit der diese durch die Vorrichtung transportiert werden, Die Zuordnung ausgewählter Volumenzusammensetzungsinformationen zu jeweiligen Schrottfragmenten kann daher einfach dadurch erfolgen, dass den Volumenzusammensetzungsinformationen eine der Reihenfolge der Schrottfragmente entsprechende Reihenfolge zugeordnet wird. Zur Sortierung der Schrottfragmente kann die Steuerungseinrichtung die Sortiereinrichtung dann abhängig von den Volumenzusammensetzungsinformationen in der vorgegebenen Reihenfolge steuern. Da die Reihenfolge der Schrottfragmente beim Transport durch die Vorrichtung möglichst unverändert bleibt, kann auf diese Weise jedes Schrottfragment entsprechend der diesem Schrottfragment jeweils zugeordneten Volumenzusammensetzungsinformation sortiert werden.

Bei einer weiteren Ausführungsform umfasst die Vorrichtung eine Erfassungseinrichtung, die dazu eingerichtet, die Lage von auf der Fördereinrichtung geförderten Schrottfragmenten zu erfassen, wobei die Steuerungseinrichtung dazu eingerichtet ist, die Analyseeinrichtung und/oder die Sortiereinrichtung abhängig von der erfassen Lage eines Schrottfragments zu steuern. Die Erfassungseinrichtung kann beispielsweise eine Kamera oder einen Laserscanner umfassen, um die Lage von Schrottfragmenten auf der Fördereinrichtung zu erfassen.

Indem die Analyseeinrichtung abhängig von der erfassten Lage des Schrottfragments gesteuert wird, kann eine zielgenaue Zusammensetzungsanalyse erfolgen. Beispielsweise kann auf diese Weise ein zur Analyse verwendeter Laserstrahl genau auf das Schrottfragment gerichtet werden.

Indem die Sortiereinrichtung abhängig von der erfassten Lage des Schrottfragments gesteuert wird, kann eine zielgenaue Sortierung erfolgen. Beispielsweise kann bei einer pneumatischen Sortierung ein Luftstoß zielgerichtet auf das Schrottfragment gerichtet werden, um es einem bestimmten Teilstrom zuzuführen.

Mit der Erfassungseinrichtung kann insbesondere auch die Reihenfolge erfasst werden, in der die Schrottfragmente durch die Vorrichtung transportiert werden. Dies erleichtert wie oben ausgeführt die Zuordnung der Volumenzusammensetzungsinformationen und die Sortierung der Schrottfragmente.

Bei einer weiteren Ausführungsform der Vorrichtung ist die Steuerungseinrichtung dazu eingerichtet, die Durchführung das zuvor beschriebene Verfahrens oder einer Ausführungsform davon zu steuern. Vorzugsweise umfasst die Steuerungseinrichtung einen Prozessor und einen damit verbundenen Speicher, der Befehle enthält, dessen Ausführung auf dem Prozessor die Durchführung des zuvor beschriebenen Verfahrens oder einer Ausführungsform davon bewirkt. Auf diese Weise ist ein im Wesentlichen automatischer Betrieb der Vorrichtung möglich, bei dem der Vorrichtung zugeführte Schrottfragmente legierungsspezifisch sortiert werden.

Weitere Vorteile und Merkmale des Verfahrens und der Vorrichtung ergeben sich aus der folgenden Beschreibung von Ausführungsbeispielen, wobei auf die beigefügte Zeichnung Bezug genommen wird.

In der Zeichnung zeigen
- Fig. 1: ein Ausführungsbeispiel der erfindungsgemäßen Vorrichtung,
- Fig. 2: die Analyseeinrichtung der Vorrichtung aus Fig. 1,
- Fig. 3: die Zuordnung einer Volumenzusammensetzungsinformation,
- Fig. 4: Beispiele für gemessene tiefenabhängige Zusammensetzungsinformationen und
- Fig. 5: ein Ausführungsbeispiel des erfindungsgemäßen Verfahrens.

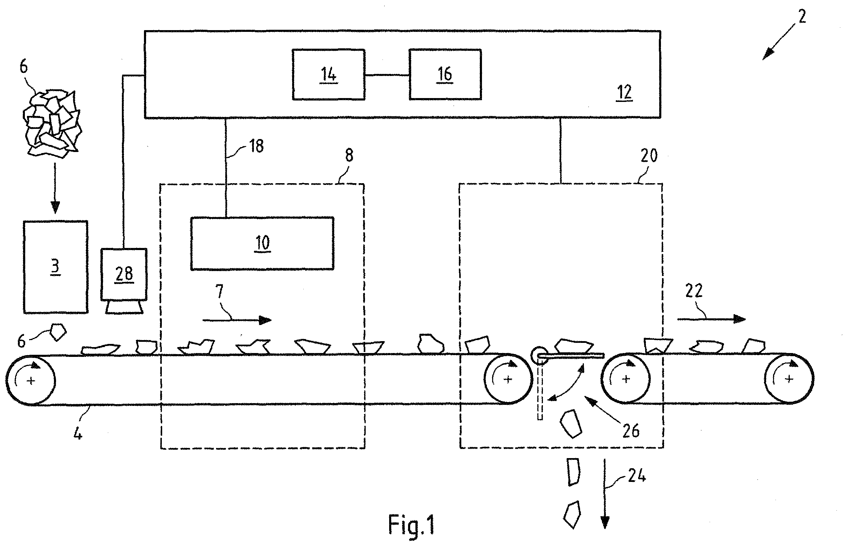

Fig. 1 zeigt ein Ausführungsbeispiel der erfindungsgemäßen Vorrichtung für die Sortierung von Metallschrott, insbesondere Aluminiumschrott, in schematischer Darstellung. Die Vorrichtung 2 umfasst eine Vereinzelungseinrichtung 3 und eine Fördereinrichtung 4 in Form eines Förderbands, mit dem von der Vereinzelungseinrichtung 3 vereinzelte Schrottfragmente 6 durch die Vorrichtung 2 gefördert werden können. Der Transport der Schrottfragmente 6 durch die Vorrichtung 2 wird in Fig. 1 durch den als Pfeil angedeuteten Materialstrom 7 repräsentiert.

Weiterhin weist die Vorrichtung 2 eine Analyseeinrichtung 8 auf, die dazu eingerichtet ist, eine Zusammensetzungsanalyse an den auf der Fördereinrichtung 4 geförderten Schrottfragmenten 6 durchzuführen. Zu diesem Zweck weist die Analyseeinrichtung 8 ein spektroskopisches Analysegerät 10 auf, bei dem es sich beispielsweise um ein Analysegerät für laserinduzierte Plasmaspektroskopie handeln kann.

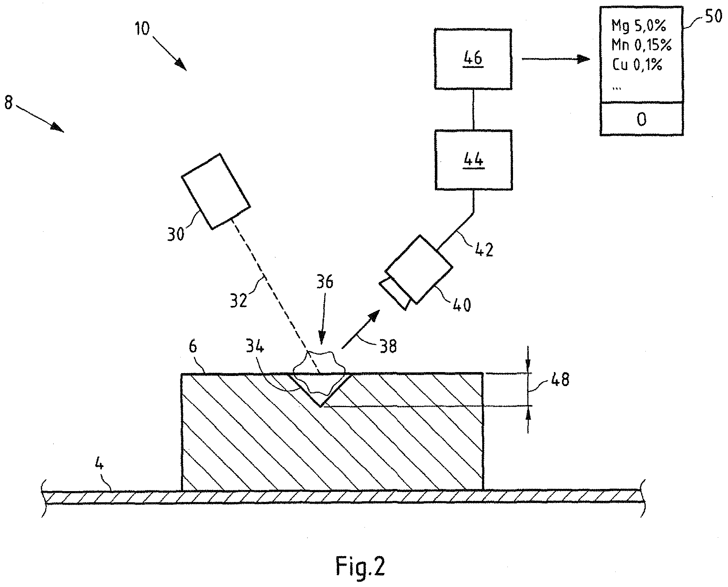

Mit dem Analysegerät 10 kann mittels einer Messung eine Oberflächenzusammensetzungsinformation über die lokale Zusammensetzung in einem Oberflächenbereich des analysierten Schrottfragments 6 bestimmt werden. Eine solche Analyse wird weiter unten im Zusammenhang mit Fig. 2 genauer erläutert.

Die Vorrichtung 2 weist weiterhin eine Steuerungseinrichtung 12 auf, die zur Steuerung der Vorrichtung 2 eingerichtet ist. Zu diesem Zweck umfasst die Steuerungseinrichtung 12 insbesondere einen Mikroprozessor 14 sowie einen damit verbundenen Speicher 16, der Befehle enthält, deren Ausführung auf dem Prozessor die Steuerung der Vorrichtung 2 veranlasst.

Das Analysegerät 10 ist über eine Datenverbindung 18 mit der Steuerungseinrichtung 12 verbunden, um die vom Analysegerät 10 bestimmte Oberflächenzusammensetzungsinformation eines Schrottfragments 6 an die Steuerungseinrichtung 12 zu übermitteln.

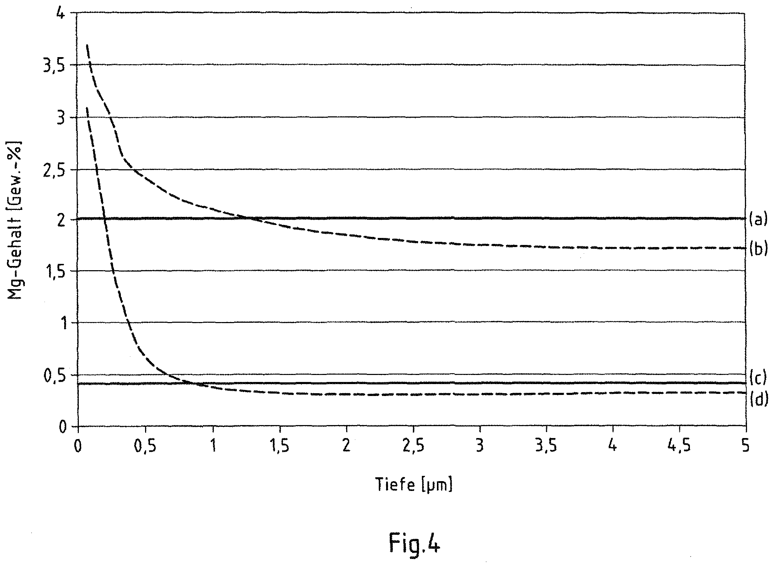

Die Steuerungseinrichtung 12 ist weiterhin dazu eingerichtet, abhängig von der über die Datenverbindung 18 empfangenen Oberflächenzusammensetzungsinformation und einer im Speicher 16 gespeicherten, vorgegebenen Zuordnungsvorschrift eine der Oberflächenzusammensetzungsinformation zugehörige Volumenzusammensetzungsinformation auszuwählen und dem vom Analysegerät 10 analysierten Schrottfragment zuzuordnen. Die Auswahl der Volumenzusammensetzungsinformation wird weiter unten im Zusammenhang mit Fig. 4 näher erläutert Die Zuordnung der Volumenzusammensetzungsinformationen zu den jeweils zugehörigen Schrottfragmenten 6 kann beispielsweise dadurch erfolgen, dass den Volumenzusammensetzungsinformationen eine der Reihenfolge der Schrottfragmente 6 entsprechende Reihenfolge zugeordnet wird. Da die Schrottfragmente 6 durch die Vereinzelungseinrichtung 3 vereinzelt werden und daher nacheinander in einer bestimmten Reihenfolge durch die Vorrichtung 2 transportiert werden, kann auf diese Weise eine eindeutige Zuordnung erreicht werden.

Die Vorrichtung 2 weist weiterhin noch eine Sortiereinrichtung 20 auf, die dazu eingerichtet ist, die Schrottfragmente 6 abhängig von der jeweils zugordneten Volumenzusammensetzungsinformation zu sortieren. Die Sortierung der Schrottfragmente 6 ist in Figur 1 schematisch durch die Aufteilung des Materialstroms 7 in zwei Teilströme 22 und 24 dargestellt. Enthält der Materialstrom 7 beispielsweise Schrottfragmente aus niedriglegierten Aluminiumlegierungen mit einem Mg- und/oder Mn-Gehalt von max. 0,5 Gew.-% und aus hochlegierten Aluminiumlegierungen, beispielsweise mit einem Mg- und/oder Mn-Gehalt von mehr als 2 Gew.-%, so kann durch die Sortiereinrichtung 20 eine Sortierung der Schrottfragmente 6 aus dem Materialstroms 7 erfolgen, bei dem Schrottfragmente, deren zugeordnete Volumenzusammensetzungsinformation einen niedrigen Mg- und Mn-Gehalt anzeigt, dem ersten Teilstrom 22 und die übrigen, hochlegierten Schrottfragmente dem zweiten Teilstrom 24 zugeordnet werden.

Um ein Schrottfragment 6 einem der beiden Teilströme 22, 24 zuzuordnen, weist die in Fig. 1 dargestellte Sortiereinrichtung 20 eine ansteuerbare, zwischen einer geschlossenen Stellung (durchgezogene Linie) und einer geöffneten Stellung (gestrichelte Linie) bewegbare Klappe 26 auf. Wenn sich die Klappe 26 in der geschlossenen Stellung befindet, wird ein über das Förderband 4 transportiertes Schrottfragment 6 dem Teilstrom 22 zugeordnet. Bei geöffneter Klappe 26 gelangt das Schrottfragment 6 hingegen zum zweiten Teilstrom 24. Die ansteuerbare Klappe 26 ist nur ein einfaches Beispiel, um den Materialstrom 7 selektiv in die Teilströme 22, 24 aufzuteilen. Stattdessen können auch andere Einrichtungen zum Sortieren der Schrottfragmente 6 vorgesehen sein. Beispielsweise können die einzelnen Schrottfragmente auch pneumatisch einem der beiden Teilströme 22, 24 zugeordnet werden, indem sie durch einem kurzen, starken Luftstoß in den jeweiligen Teilstrom befördert werden.

Weiterhin weist die Vorrichtung 2 noch eine Erfassungseinrichtung 28 in Form einer Kamera auf, mit der die Lage von auf der Fördereinrichtung 4 geförderten Schrottfragmenten 6 erfasst werden kann. Anstelle einer Kamera kann beispielsweise auch ein Laserscanner verwendet werden.

Im Folgenden wird der Betrieb der Vorrichtung 2 beschrieben:
Eine Menge Schrottfragmente 6 wird in die Vereinzelungseinrichtung 3 gegeben und dort vereinzelt, so dass die Schrottfragmente 6 nacheinander in einer festgelegten Reihenfolge auf die Fördereinrichtung 4 gelangen und mit dieser durch die Vorrichtung transportiert werden.

Die vereinzelten Schrottfragmente 6 werden nacheinander mit der Erfassungseinrichtung 28 erfasst, wodurch die Reihenfolge der Schrottfragmente 6 erfasst wird.

Die einzelnen Schrottfragmente werden dann in der Analysevorrichtung 8 analysiert und die Steuerungseinrichtung 12 wählt anhand der bei der Analyse ermittelten Oberflächenzusammensetzungsinformation und der vorgegebenen Zuordnungsvorschrift eine zugehörige Volumenzusammensetzungsinformation aus und ordnet diese dem jeweiligen Schrottfragment 6 zu. Die Zuordnung der Volumenzusammensetzungsinformationen erfolgt vorliegend dadurch, dass den Volumenzusammensetzungsinformationen die erfasste Reihenfolge der Schrottfragmente 6 zugeordnet wird.

In der Sortiereinrichtung 20 werden die Schrottfragmente 6 dann abhängig von der jeweils zugeordneten Volumenzusammensetzungsinformation je einem der beiden Teilströme 22, 24 zugeführt. Zu diesem Zweck wird anhand der mit der Erfassungseinrichtung 28 erfassten Reihenfolge der Schrottfragmente 6 bestimmt, welches Schrottfragment 6 als nächstes zur Klappe 26 gelangt und die Klappe 26 abhängig von der diesem Schrottfragment 6 zugeordneten Volumenzusammensetzungsinformation gesteuert, so dass das Schrottfragment dem richtigen Teilstrom 22, 24 zugeführt wird.

Auf diese Weise ist eine effektive Trennung verschiedener Aluminiumlegierungen aus dem Materialstrom 7 möglich. Die Trennung auf Grundlage der den Schottfragmenten jeweils zugeordneten Volumenzusammensetzungsinformation vermeidet Fehlinterpretationen und erlaubt dadurch eine saubere Sortierung der einzelnen Schrottfragmente.

In Fig. 1 sind exemplarisch zwei Teilströme 22, 24 dargestellt. Der Materialstrom 7 kann aber natürlich auch in mehr als zwei Teilströme aufgeteilt werden.

Die Sortiereinrichtung 20 ist in Fig. 1 als räumlich separate Einheit zur Analyseeinrichtung 8 dargestellt. Die Sortiereinrichtung 20 und die Analyseeinrichtung 8 können jedoch auch räumlich überlappen. Beispielsweise kann die Sortierung der Schrottfragmente 8 unmittelbar nach der Analyse erfolgen.

Fig. 2 zeigt eine schematische Darstellung der Analyseeinrichtung 8 der Vorrichtung 2 aus Fig. 1. Bei dem Analysegerät 10 handelt es sich in diesem Ausführungsbeispiel um ein Analysegerät für laserinduzierte Plasmaspektroskopie. Das Analysegerät 10 umfasst eine Laserquelle 30, mit der ein auf dem Förderband 4 befördertes Schrottfragment 6 mit einem gepulsten Laserstrahl 32 beaufschlagt werden kann. Durch den auftreffenden Laserstrahl 32 wird ein kleines Volumen 34 an der Oberfläche des Schrottfragments 6 verdampft und zu einem Plasma 36 ionisiert. In der Schrottfragmentoberfläche entsteht dadurch ein entsprechender Krater. Beim Zerfall des Plasmas 36 wird Licht 38 emittiert, das charakteristisch für die im Volumen 34 enthaltenen Legierungselemente ist. Das Volumen 34 bzw. der zu dem Volumen korrespondierende Krater weisen in diesem Ausführungsbeispiel eine Kegelform auf. Der Querschnitt des Kraters bzw. des Volumens 34 nimmt also in der Tiefe ab, so dass das Licht 38 vor allem von den obersten Schichten des Volumens 34 dominiert wird.

Das Analysegerät 10 weist eine Optik 40 auf, um das Licht 38 einzufangen und über einen Lichtleiter 42 an ein Spektrometer 44 weiterzuleiten, mit dem die Spektralverteilung des Lichts 38 analysiert werden kann. Eine an das Spektrometer angeschlossene Auswerteeinrichtung 46 berechnet dann aus der gemessenen Spektralverteilung die Zusammensetzung des Volumens 34. Da der Laserstrahl 32 nur eine bestimmte Eindringtiefe 48 hat, die abhängig von der eingestellten Laserleistung typischerweise im Bereich von 1 bis 10 µm liegt, liefert die Auswerteeinrichtung 46 eine Oberflächenzusammensetzungsinformation 50, d.h. eine Zusammensetzungsinformation über ein oberflächennahes Volumen des Schrottfragment-Materials. Die Oberflächenzusammensetzungsinformation 50 ist in Fig. 2 mit einem "O" für "Oberfläche" gekennzeichnet und enthält Gehalte für verschiedene Legierungselemente (Mg, Mn, Cu etc.) in Gew.-%. Diese Oberflächenzusammensetzungsinformation 50 wird bei der Vorrichtung 2 zur weiteren Verarbeitung über die Datenverbindung 18 an die Steuerungseinrichtung 12 gesendet.

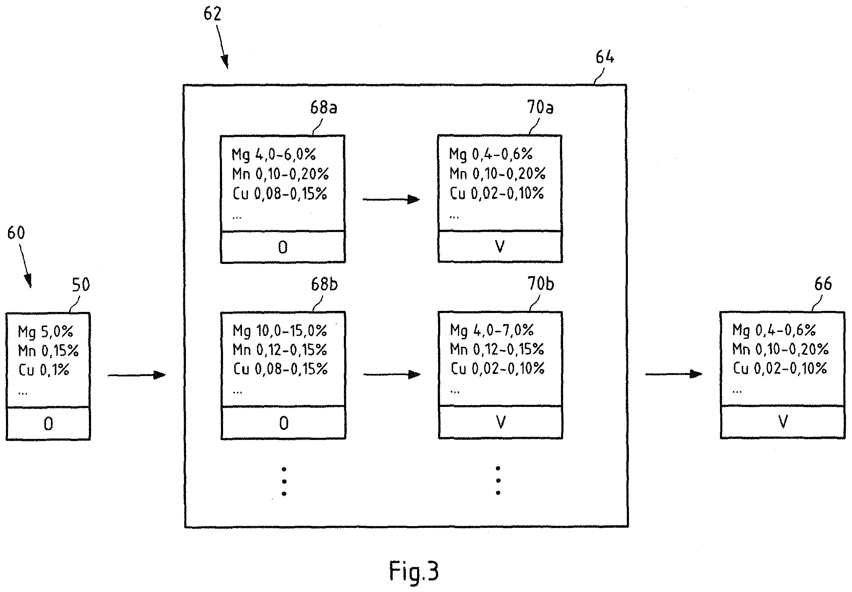

Fig. 3 zeigt schematisch, wie die Steuerungseinrichtung 12 der von der Analyseeinrichtung 8 gemessenen Oberflächenzusammensetzungsinformation 50 eine zugehörige Volumenzusammensetzungsinformation zuordnet.

Die Steuerungseinrichtung 12 empfängt zunächst in einem ersten Schritt 60 die von der Analyseeinrichtung 8 gemessene Oberflächenzusammensetzungsinformation 50.

In einem zweiten Schritt 62 wählt die Steuerungseinrichtung 12 abhängig von der Oberflächenzusammensetzungsinformation 50 und einer im Speicher 16 gespeicherten Zuordnungsvorschrift 64 die zugehörige Volumenzusammensetzungsinformation 66 aus. Zu diesem Zweck umfasst die Zuordnungsvorschrift 64 im vorliegenden Beispiel eine Tabelle, in der mehreren Legierungsbereichen für die Oberflächenzusammensetzungsinformation 68a, 68b etc. jeweils ein zugehöriger Legierungsbereich für die Volumenzusammensetzungsinformation 70a, 70b etc. zugeordnet ist. Die in der Figur zahlenmäßig dargestellten Legierungsbereiche sind exemplarisch.

Die Steuerungseinrichtung 12 vergleicht die Oberflächenzusammensetzungsinformation 50 mit den Legierungsbereichen für die Oberflächenzusammensetzungsinformation 68a, 68b und wählt aus diesen den passenden Legierungsbereich für die Oberflächenzusammensetzungsinformation aus, im vorliegenden Beispiel den Legierungsbereich 68a. Der diesem Legierungsbereich zugeordnete Legierungsbereich für die Volumenzusammensetzungsinformation 70a stellt dann die der Oberflächenzusammensetzungsinformation 50 zugeordnete Volumenzusammensetzungsinformation 66 dar, mit der die Sortiereinrichtung 20 gesteuert wird.

Durch die Auswahl einer zugehörigen Volumenzusammensetzungsinformation 66 für die Oberflächenzusammensetzungsinformation 50 und die Steuerung der Sortiereinrichtung 20 abhängig von der Volumenzusammensetzungsinformation 66 anstelle der Oberflächenzusammensetzungsinformation 50 wird berücksichtigt, dass die mit der Analyseeinrichtung 8 bestimmte Zusammensetzung an der Oberfläche des Schrottfragments 6 aufgrund von Seigerungs- und Diffusionseffekten von der tatsächlichen Volumenzusammensetzung des Schrottfragments 6 abweicht. Dadurch ist eine zuverlässigere legierungsspezifische Sortierung der Schrottfragmente auf Basis der Volumenzusammensetzung möglich.

Zur Festlegung der Zuordnungsvorschrift, z.B. für eine zu sortierende Schrottlieferung, wird vorzugsweise untersucht, welche Volumenzusammensetzungen eines Schrottfragments sich in welchen Oberflächenzusammensetzungen niederschlagen. Beispielweise können einer Menge Schrottfragmente, wie einer zu sortierenden Schrottlieferung, vor dem Zuführen zu der Vorrichtung 2 einzelne Probenfragmente entnommen werden, für die einerseits die Oberflächenzusammensetzung und andererseits die Volumenzusammensetzung analysiert wird. Aus den bei dieser Analyse ermittelten Zusammenhängen zwischen der Oberflächenzusammensetzung und der Volumenzusammensetzung kann dann die Zuordnungsvorschrift 64 definiert werden.

Die Analyse der Probenfragmente kann beispielsweise mittels Glimmentladungsspektroskopie (GDOES) untersucht werden. Bei diesem Verfahren wird das Probenfragment in einem Gleichspannungsplasma als Kathode benutzt. Durch Kathodenzerstäubung wird nach und nach schichtweise Material von der Oberfläche des Probenfragments abgetragen, wobei die abgetragenen Atome im Plasma charakteristisches Licht emittieren, das spektroskopisch untersucht wird. Auf diese Weise kann die Zusammensetzung der Probenfragmente abhängig von der Tiefe analysiert werden.

Fig. 4 zeigt ein Diagramm mit Messergebnissen einer GDOES-Analyse an zwei Schrottfragmenten. Das Diagramm zeigt die von der Tiefe abhängige Zusammensetzung der Schrottfragmente am Beispiel des Legierungselements Mg. Die Abszisse gibt die Tiefe in µm an, d.h. den Abstand von der Oberfläche des Schrottfragments in das Volumen des Schrottfragments. Die Ordinate gibt den lokalen Mg-Gehalt in Gew.-% in der entsprechenden Tiefe an.

Das erste analysierte Schrottfragment bestand aus einer Aluminiumlegierung vom Typ AA5XXX. Die horizontale Linie (a) zeigt den durchschnittlichen Mg-Gehalt des Schrottfragments, der mittels optischer Emissionsspektroskopie (OES) bestimmt wurde. Bei der OES dringen die Funken tiefer in das Material ein und liefert somit einen Wert, der der durchschnittlichen Zusammensetzung entspricht. Typischerweise kann mit einer OES ein sehr genauer Wert für die Zusammensetzung im Volumen gemessen werden. Die Messkurve (b) zeigt den von der Tiefe abhängigen Mg-Gehalt des Schrottfragments, der mittels GDOES bestimmt wurde.

Das zweite analysierte Schrottfragment bestand aus einer Aluminiumlegierung vom Typ AA6XXX. Die horizontale Linie (c) zeigt wiederum den durchschnittlichen Mg-Gehalt des Schrottfragments, der mittels optischer Emissionsspektroskopie (OES) bestimmt wurde. Die Messkurve (d) zeigt den von der Tiefe abhängigen Mg-Gehalt des Schrottfragments, der mittels GDOES bestimmt wurde.

Aus dem Diagramm ist ersichtlich, dass der Mg-Gehalt unmittelbar an der Oberfläche der Schrottfragmente (bei 0 - ca. 0,5 µm) durch Seigerungs- und Diffusionseffekte stark erhöht ist und deutlich über dem jeweiligen durchschnittlichen Mg-Gehalt liegt. Mit größerer Tiefe fällt der Mg-Gehalt stark ab und liegt im untersuchten Tiefenbereich von ca. 0,5 bis 5 µm sogar unterhalb des durchschnittlichen Mg-Gehalts, da Mg von hier an die Oberfläche diffundiert ist.

Die in Fig. 4 dargestellten Messergebnisse können beispielsweise verwendet werden, um eine entsprechende Zuordnungsvorschrift 64, zum Beispiel für eine zu sortierende Schrottlieferung, zu definieren. Insbesondere kann aus dem Diagramm abgeleitet werden, welchem Mg-Gehalt im Volumen bei bestimmten Schrottfragmenten ein bestimmter Mg-Gehalt einer Oberflächenzusammensetzungsinformation entspricht.

Durch eine Integral- oder Mittelwertbildung des tiefenabhängigen Mg-Gehalts aus Kurve (b) bzw. (d) im Tiefenbereich von 0 µm bis zur Eindringtiefe 48 des Laserstrahls 32 lässt sich der Mg-Gehalt bestimmen, der sich bei dem Volumengehalt nach Linie (a) bzw. (c) aus der Messung mit der in Fig. 2 dargestellten Analyseeinrichtung 8 ergibt. Die Eindringtiefe 48 des Laserstrahls 32 hängt im Wesentlichen von der Laserleistung ab und ist damit bei fest eingestellter Laserleistung bekannt. Ist die Eindringtiefe 48 durch Einstellen der Laserleistung zum Beispiel auf 5 µm festgelegt, so kann der Mg-Wert einer Oberflächenzusammensetzungsinformation insbesondere durch Mittelung der Gehalte zwischen 0 und 5 µm aus der GDOES-Analyse berechnet werden. Vorzugsweise erfolgt die Mittelung bzw. Integration gewichtet, um den Footprint des Laserstrahls und/oder die Kraterform des vom Laserstrahl in die Schrottfragmentoberfläche eingebrachten Kraters mitberücksichtigen zu können. Bei dem in Fig. 2 dargestellten kegelförmigen Krater werden zum Beispiel bevorzugt oberflächennähere Gehalte entsprechend stärker gewichtet.

Durch diese und entsprechende Analysen für weitere Legierungselemente lassen sich damit die Legierungsbereiche für die Oberflächenzusammensetzungsinformation 68a, 68b etc. und die zugehörigen Legierungsbereiche für die Volumenzusammensetzungsinformation 70a, 70b etc. der Zuordnungsvorschrift 64 festlegen.

Das Diagramm in Fig. 4 zeigt weiterhin auch, dass eine direkte Verwendung der von der Analyseeinrichtung 8 gemessenen Mg-Gehalte zur Steuerung der Sortiereinrichtung 20 ohne Berücksichtigung der Zuordnungsvorschrift 64 zu erheblichen Fehlinterpretationen hinsichtlich der Zusammensetzung der Schrottfragmente führen würde.

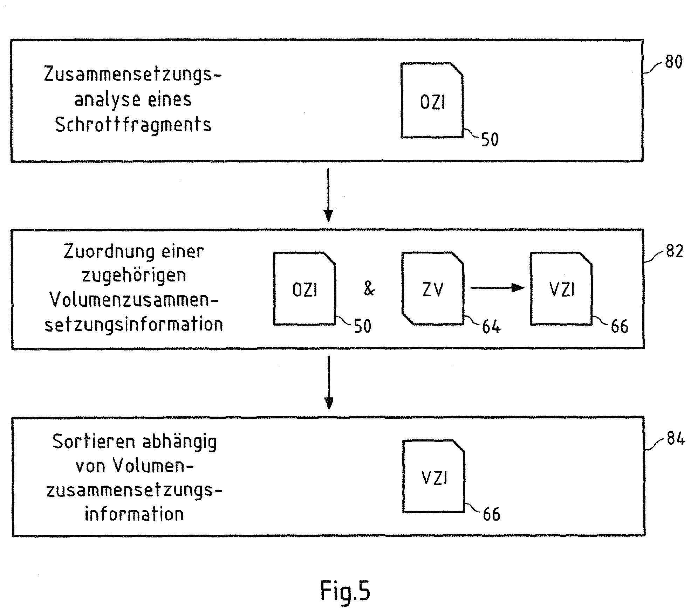

Fig. 5 zeigt ein Ausführungsbeispiel eines erfindungsgemäßen Verfahrens unter Verwendung der in Fig. 1 dargestellten Vorrichtung 2.

In einem ersten Schritt 80 des Verfahrens werden Schrottfragmente 6 mit der Fördereinrichtung 4 der Analyseeinrichtung 8 zugeführt und dort einer Zusammensetzungsanalyse unterzogen. Zu diesem Zweck werden die einzelnen Schrottfragmente von dem Analysegerät 10 mit einem gepulsten Laserstrahl 32 beaufschlagt und aus der resultierenden Lichtemission eine jeweilige Oberflächenzusammensetzungsinformation 50 bestimmt.

In einem zweiten Schritt 82 des Verfahrens wählt die Steuerungseinrichtung 12 für jede Oberflächenzusammensetzungsinformation 50 mittels der Zuordnungsvorschrift 64 eine zugehörige Volumenzusammensetzungsinformation 66 aus und ordnet diese dem jeweiligen Schrottfragment 6 zu.

In einem dritten Schritt 84 steuert die Steuerungseinrichtung 12 die Sortiereinrichtung 20 derart an, dass das jeweilige Schrottfragment 6 abhängig von der Volumenzusammensetzungsinformation 66 sortiert wird, d.h. im vorliegenden Beispiel einem der beiden Teilströme 22 oder 24 zugeordnet wird.

Wie sich aus den zuvor beschriebenen Ausführungsbeispielen ergibt, lässt sich mit der beschriebenen Vorrichtung und mit dem beschriebenen Verfahren damit eine verbesserte legierungsspezifische Sortierung von Schrottfragmenten erreichen.

## Patentansprüche

1. Verfahren für das legierungsabhängige Sortieren von Metallschrott, insbesondere Aluminiumschrott,
- bei dem an einem Schrottfragment (6) eine Zusammensetzungsanalyse durchgeführt wird, wobei mittels einer Messung an dem Schrottfragment (6) eine Oberflächenzusammensetzungsinformation (50) über die lokale Zusammensetzung in einem Oberflächenbereich des Schrottfragments (6) bestimmt wird,
**dadurch gekennzeichnet,**
- **dass** dem Schrottfragment (6) abhängig von der mittels Messung bestimmten Oberflächenzusammensetzungsinformation (50) und einer vorgegebenen Zuordnungsvorschrift (64) eine zugehörige Volumenzusammensetzungsinformation (66) über die Zusammensetzung des Schrottfragments (6) im Volumen zugeordnet wird.

2. Verfahren nach Anspruch 1,
- bei dem eine Menge von Schrottfragmenten (6) bereitgestellt wird,
- bei dem für mehrere Schrottfragmente (6) aus der Menge von Schrottfragmenten jeweils eine Zusammensetzungsanalyse durchgeführt wird, wobei mittels einer Messung an dem jeweiligen Schrottfragment (6) eine Oberflächenzusammensetzungsinformation (50) über die lokale Zusammensetzung in einem Oberflächenbereich des jeweiligen Schrottfragments (6) bestimmt wird, und
- bei dem dem jeweiligen Schrottfragment (6) abhängig von der mittels Messung bestimmten Oberflächenzusammensetzungsinformation (50) und einer vorgegebenen Zuordnungsvorschrift (64) eine zugehörige Volumenzusammensetzungsinformation (66) über die Zusammensetzung des jeweiligen Schrottfragments (6) im Volumen zugeordnet wird.

3. Verfahren nach Anspruch 1 oder 2,
**dadurch gekennzeichnet, dass** das Schrottfragment (6) abhängig von der zugeordneten Volumenzusammensetzungsinformation (66) sortiert wird.

4. Verfahren nach einem der Ansprüche 1 bis 3,
**dadurch gekennzeichnet, dass** dem Schrottfragment (6) abhängig von der mittels Messung bestimmten Oberflächenzusammensetzungsinformation (50) und einer vorgegebenen Zuordnungsvorschrift (64) eine zugehörige Volumenzusammensetzungsinformation (66) zugeordnet wird, indem abhängig von der mittels Messung bestimmten Oberflächenzusammensetzungsinformation (50) und der vorgegebenen Zuordnungsvorschrift (64) eine Volumenzusammensetzungsinformation (66) aus einer Mehrzahl vorgegebener Volumenzusammensetzungsinformationen (70a-b) ausgewählt wird.

5. Verfahren nach Anspruch 4,
**dadurch gekennzeichnet, dass** den vorgegebenen Volumenzusammensetzungsinformationen (70a-b) jeweils eine vorgegebene zugehörige Oberflächenzusammensetzungsinformation (68a-b) zugeordnet ist und die Auswahl der Volumenzusammensetzungsinformation (66) aus der Mehrzahl vorgegebener Volumenzusammensetzungsinformationen (70a-b) durch einen Vergleich der mittels Messung bestimmten Oberflächenzusammensetzungsinformation (50) mit den vorgegebenen Oberflächenzusammensetzungsinformationen (68a-b) erfolgt.

6. Verfahren nach einem der Ansprüche 1 bis 5,
**dadurch gekennzeichnet, dass** bei der Zusammensetzungsanalyse eine Oberflächenzusammensetzungsinformation (50) über die lokale Zusammensetzung in einem Oberflächenbereich des Schrottfragments (6) bestimmt wird, wobei sich der Oberflächenbereich von der Oberfläche des Schrottfragments (6) bis in eine vorbekannte Tiefe, insbesondere bis in eine Tiefe im Bereich vom 2-10 µm, erstreckt.

7. Verfahren nach einem der Ansprüche 1 bis 6,
**dadurch gekennzeichnet, dass** die Zusammensetzungsanalyse eine spektroskopische Analyse umfasst, insbesondere eine laserinduzierte Plasmaspektroskopie (LIBS) oder eine Röntgenfluoreszenzanalyse (XRF).

8. Verfahren nach einem der Ansprüche 1 bis 7,
**dadurch gekennzeichnet, dass** die durch Messung bestimmte Oberflächenzusammensetzungsinformation (50) Werte für die Gehalte von mindestens zwei Legierungskomponenten des Schrottfragments umfasst.

9. Verfahren nach einem der Ansprüche 2 bis 8,
**dadurch gekennzeichnet, dass** die Schrottfragmente (6) vereinzelt werden, bevor an den Schrottfragmenten (6) eine Zusammensetzungsanalyse durchgeführt wird.

10. Vorrichtung (2) für die Sortierung von Metallschrott, insbesondere Aluminiumschrott, vorzugsweise zur Durchführung des Verfahrens nach einem der Ansprüche 1 bis 9,
- mit einer Fördereinrichtung (4), eingerichtet zur Förderung einer Menge Schrottfragmente, und
- mit einer Analyseeinrichtung (8), eingerichtet zur Durchführung von Zusammensetzungsanalysen von auf der Fördereinrichtung (4) geförderten Schrottfragmenten (6), wobei eine Zusammensetzungsanalyse eines Schrottfragments (6) die Bestimmung einer Oberflächenzusammensetzungsinformation (50) über die lokale Zusammensetzung in einem Oberflächenbereich des Schrottfragments (6) mittels einer Messung umfasst,
**dadurch gekennzeichnet,**
- **dass** die Vorrichtung (2) eine Steuerungseinrichtung (12) aufweist, die dazu eingerichtet ist, den mit der Analyseeinrichtung (8) analysierten Schrottfragmenten (6) abhängig von der mittels Messung bestimmten Oberflächenzusammensetzungsinformation (50) und einer vorgegebenen Zuordnungsvorschrift (64) jeweils eine zugehörige Volumenzusammensetzungsinformation (66) über die Zusammensetzung des Schrottfragments (6) im Volumen zuzuordnen.

11. Vorrichtung nach Anspruch 10, weiter umfassend eine Sortiereinrichtung (20), die dazu eingerichtet ist, Schrottfragmente (6) abhängig von der den Schrottfragmenten (6) durch die Steuerungseinrichtung (12) jeweils zugeordneten Volumenzusammensetzungsinformation (66) zu sortieren.

12. Vorrichtung nach Anspruch 10 oder 11,
**dadurch gekennzeichnet, dass** die Analyseeinrichtung (8) ein spektroskopisches Analysegerät (10) umfasst, insbesondere ein Analysegerät für laserinduzierte Plasmaspektroskopie (LIBS) oder Röntgenfluoreszenzanalyse (XRF).

13. Vorrichtung nach einem der Ansprüche 10 bis 12, weiter umfassend eine Vereinzelungseinrichtung, die dazu eingerichtet ist, Schrottfragmente (6) zu vereinzeln, bevor sie der Analyseeinrichtung (8) zugeführt werden.

14. Vorrichtung nach einem der Ansprüche 10 bis 13, weiter umfassend eine Erfassungseinrichtung (28), die dazu eingerichtet, die Lage von auf der Fördereinrichtung (4) geförderten Schrottfragmenten (6) zu erfassen, wobei die Steuerungseinrichtung dazu eingerichtet ist, die Analyseeinrichtung (8) und/oder die Sortiereinrichtung (12) abhängig von der erfassen Lage eines Schrottfragments (6) zu steuern.

15. Vorrichtung nach einem der Ansprüche 10 bis 14, **dadurch gekennzeichnet, dass** die Steuerungseinrichtung (12) dazu eingerichtet ist, die Durchführung eines Verfahrens nach einem der Ansprüche 1 bis 9 zu steuern.

## Claims

1. Method for the alloy-dependent sorting of scrap metal, in particular aluminum scrap,
- in which a composition analysis is performed on a scrap fragment (6), wherein surface composition information (50) about the local composition in a surface region of the scrap fragment (6) is determined by measurement on the scrap fragment (6),
**characterized in,**
- **that** associated bulk composition information (66) about the composition of the scrap fragment (6) in the bulk is assigned to the scrap fragment (6) as a function of the surface composition information (50) determined by measurement and a predetermined assignment rule (64).

2. Method according to claim 1,
- in which a quantity of scrap fragments (6) is provided,
- in which a composition analysis is respectively carried out on a plurality of scrap fragments (6) from the quantity of scrap fragments, wherein surface composition information (50) about the local composition in a surface region of the respective scrap fragment (6) is determined by means of measurement on the respective scrap fragment (6), and
- in which associated bulk composition information (66) about the composition of the respective scrap fragment (6) in the bulk is assigned to the respective scrap fragment (6) as a function of the surface composition information (50) determined by measurement and a predetermined assignment rule (64).

3. Method according to claim 1 or 2,
**characterized in that** the scrap fragment (6) is sorted as a function of the associated bulk composition information (66).

4. Method according to any one of the claims 1 to 3,
**characterized in that** associated bulk composition information (66) is assigned to the scrap fragment (6) as a function of the surface composition information (50) determined by measurement and a predetermined assignment rule (64) **in that** bulk composition information (66) is selected from a plurality of predetermined pieces of bulk composition information (70a-b) as a function of the surface composition information (50) determined by means of measurement and the predetermined assignment rule (64).

5. Method according to claim 4,
**characterized in that** a predetermined piece of surface composition information (68a-b) is respectively assigned to the predetermined piece of bulk composition information (70a-b) and the selection of the piece of bulk composition information (66) from the plurality of predetermined pieces of bulk composition information (70a-b) takes place by a comparison of the measured surface composition information (50) with the predetermined pieces of surface composition information (68a-b).

6. Method according to any one of the claims 1 to 5,
**characterized in that** in the composition analysis, surface composition information (50) about the local composition in a surface region of the scrap fragment (6) is determined, wherein the surface region extends from the surface of the scrap fragment (6) to a known depth, in particular to a depth in the range of 2 -10 µm.

7. Method according to any one of the claims 1 to 6,
**characterized in that** the composition analysis comprises a spectroscopic analysis, in particular laser-induced breakdown spectroscopy (LIBS) or X-ray fluorescence analysis (XRF).

8. Method according to any one of the claims 1 to 7,
**characterized in that** the surface composition information (50) determined by measurement comprises values for the contents of at least two alloy components of the scrap fragment.

9. Method according to any one of the claims 2 to 8,
**characterized in that** the scrap fragments (6) are separated before a composition analysis is performed on the scrap fragments (6).

10. Apparatus (2) for the sorting of metal scrap, in particular aluminum scrap, preferably for carrying out the method according to one of the claims 1 to 9,
- with a conveyor (4) configured to convey a quantity of scrap fragments and
- with an analysis device (8) configured to perform composition analyses of scrap fragments (6) conveyed on the conveyor (4), wherein composition analysis of a scrap fragment (6) comprises determination of surface composition information (50) about the local composition in a surface region of the scrap fragment (6) by means of measurement,
**characterized in**
- **that** the apparatus (2) comprises a control device (12) which is configured to respectively assign associated bulk composition information (66) about the composition of the scrap fragment (6) in the bulk to the scrap fragments (6) analyzed by the analysis device (8) as a function of the surface composition information (50) determined by measurement and a predetermined assignment rule (64).

11. Apparatus according to claim 10, further comprising a sorting device (20) which is configured to sort scrap fragments (6) as a function of the bulk composition information (66) respectively assigned to the scrap fragments (6) by the control device (12).

12. Apparatus according to claim 10 or 11, **characterized in that** the analysis device (8) comprises a spectroscopic analysis device (10), in particular an analysis device for laser-induced breakdown spectroscopy (LIBS) or X-ray fluorescence analysis (XRF).

13. Apparatus according to one of the claims 10 to 12, further comprising a separating device which is configured to separate scrap fragments (6) before they are fed to the analysis device (8).

14. Apparatus according to one of the claims 10 to 13, further comprising a detection device (28) which is configured to detect the position of scrap fragments (6) conveyed on the conveyor (4), wherein the control device is configured to control the analysis device (8) and/or the sorting device (12) as a function of the detected position of a scrap fragment (6).

15. Apparatus according to one of the claims 10 to 14, **characterized in that** the control device (12) is configured to control the implementation of a method according to one of the claims 1 to 9.

## Revendications

1. Procédé pour le tri de déchets métalliques, en particulier de déchets d'aluminium, en fonction de l'alliage,
- selon lequel une analyse de composition est effectuée sur un fragment de déchet (6), où une information de composition de surface (50) relative à la composition locale dans une zone de surface du fragment de déchet (6) est déterminée au moyen d'une mesure sur le fragment de déchet (6),
**caractérisé**
- **en ce qu'**une information de composition de volume (66) correspondante relative à la composition du fragment de déchet (6) dans le volume est associée au fragment de déchet (6) en fonction de l'information de composition de surface (50) déterminée au moyen de la mesure et d'une règle de correspondance (64) prédéfinie.

2. Procédé selon la revendication 1,
- selon lequel une quantité de fragments de déchets (6) est mise à disposition,
- selon lequel respectivement une analyse de composition est effectuée pour plusieurs fragments de déchets (6) de la quantité de fragments de déchets, où une information de composition de surface (50) relative à la composition locale dans une zone de surface du fragment de déchet (6) respectif est déterminée au moyen d'une mesure sur le fragment de déchet (6), et
- selon lequel une information de composition de volume (66) correspondante relative à la composition du fragment de déchet (6) respectif dans le volume est associée au fragment de déchet (6) respectif en fonction de l'information de composition de surface (50) déterminée au moyen de la mesure et d'une règle de correspondance (64) prédéfinie.

3. Procédé selon la revendication 1 ou 2,
**caractérisé en ce que** le fragment de déchet (6) est trié en fonction de l'information de composition de volume (66) associée.

4. Procédé selon l'une des revendications 1 à 3,
**caractérisé en ce qu'**une information de composition de volume (66) correspondante est associée au fragment de déchet (6) en fonction de l'information de composition de surface (50) déterminée au moyen de la mesure et d'une règle de correspondance (64) prédéfinie, et ce en sélectionnant une information de composition de volume (66) parmi une pluralité d'informations de composition de volume (70a-b) prédéfinies en fonction de l'information de composition de surface (50) déterminée au moyen de la mesure et d'une règle de correspondance (64) prédéfinie.

5. Procédé selon la revendication 4,
**caractérisé en ce qu'**aux informations de composition de volume (70a-b) prédéfinies, une information de composition de surface (68a-b) correspondante prédéfinie soit respectivement associée, et la sélection de l'information de composition de volume (66) à partir de la plutalite d'informations de composition de volume (70a-b) prédéfinie a lieu par une comparaison de l'information de composition de surface (50) déterminée au moyen de la mesure avec l'information de composition de surface (68a-b) prédéfinie.

6. Procédé selon l'une des revendications 1 à 5,
**caractérisé en ce que**, lors de l'analyse de composition, une information de composition de surface (50) relative à la composition locale dans une zone de surface du fragment de déchet (6) est déterminée, la zone de surface s'étendant de la surface du fragment de déchet (6) jusqu'à une profondeur déjà connue, notamment jusqu'à une profondeur se situant dans une plage allant de 2 à 10 µm.

7. Procédé selon l'une des revendications 1 à 6.
**caractérisé en ce que** l'analyse de composition comporte une analyse spectroscopique, en particulier une spectrométrie d'émission atomique de plasma induit par laser (LIBS) ou une analyse par fluorescence X (XRF).

8. Procédé selon l'une des revendications 1 à 7,
**caractérisé en ce que** l'information de composition de surface (50) déterminée au moyen de la mesure comporte des valeurs pour la teneur d'au moins deux composants d'alliage du fragment de déchet.

9. Procédé selon l'une des revendications 2 à 8,
**caractérisé en ce que** les fragments de déchets (6) sont séparés les uns des autres avant d'effectuer une analyse de composition sur les fragments de déchets (6).

10. Dispositif (2) pour le tri de déchets métalliques, en particulier de déchets d'aluminium, de préférence pour la mise en œuvre du processus selon l'une des revendications 1 à 9,
- avec un dispositif de transport (4) conçu pour transporter une quantité de fragments de déchets, et
- avec un dispositif d'analyse (8) conçu pour la mise en œuvre d'analyses de composition de fragments de déchets (6) transportés sur le dispositif de transport (4), une analyse de composition d'un fragment de déchet (6) comportant la détermination d'une information de composition de surface (50) relative à la composition locale dans une zone de surface du fragment de déchet (6) au moyen d'une mesure,
**caractérisé en ce que**
le dispositif (2) présente un dispositif de commande (12) qui est conçu pour associer aux fragments de déchets (6) analysés à l'aide du dispositif d'analyse (8) respectivement une information de composition de volume (66) correspondante relative à la composition du fragment de déchet (6) dans le volume, en fonction de l'information de composition de surface (50) déterminée au moyen de la mesure et d'une règle de correspondance (64) prédéfinie.

11. Dispositif selon la revendication 10, comportant en outre un dispositif de tri (20) qui est conçu pour trier des fragments de déchets (6) en fonction de l'information de composition de volume (66) respectivement associée aux fragments de déchets (6) par le dispositif de commande (12).

12. Dispositif selon la revendication 10 ou 11,
**caractérisé en ce que** le dispositif d'analyse (8) comporte un appareil d'analyse spectroscopique (10), notamment un appareil d'analyse pour une spectrométrie d'émission atomique de plasma induit par laser (LIBS) ou une analyse par fluorescence aux rayons X (XRF).

13. Dispositif selon l'une des revendications 10 à 12, comportant en outre un dispositif de séparation qui est conçu pour séparer des fragments de déchets (6) les uns des autres avant de les amener au dispositif d'analyse (8).

14. Dispositif selon l'une des revendications 10 à 13, comportant en outre un dispositif de détection (28) qui est conçu pour détecter la position des fragments de déchets (6) transportés sur le dispositif de transport (4), le dispositif de commande étant conçu pour commander le dispositif d'analyse (8) et/ou le dispositif de tri (12) en fonction de la position détectée d'un fragment de déchet (6).

15. Dispositif selon l'une des revendications 10 à 14,
**caractérisé en ce que** le dispositif de commande (12) est conçu pour commander la mise en œuvre d'un processus selon l'une des revendications 1 à 9.
